# FASCICULE DE BREVET EUROPEEN

(11) **EP 0 681 831 B1**
(45) Date de publication et mention de la délivrance du brevet: **19.12.2001**
(21) Numéro de dépôt: 95401077.3
(22) Date de dépôt: 10.05.1995
(51) Int. Cl.: A61K 7/48

(54) **Utilisation d'extraits de bactéries filamenteuses comme agents cosmétiques contre le vieillissement cutané**
Verwendung von Extracten aus filamentösen Bakterien als kosmetische Mittel gegen Hautaltern
Use of extracts of filamentous bacteria as cosmetic agents against skin aging

(30) Priorité: 10.05.1994 FR 9405741
(43) Date de publication de la demande: 15.11.1995
(73) Titulaire: L'OREAL, 75008 Paris (FR)
(72) Inventeur: Aubert, Lucien, Les Rocailles, F-06320 Cap D'Ail (FR); Martin, Richard, Roche Corbon, F-37290 Vouvray (FR)
(74) Mandataire: Tonnellier, Jean-Claude

(56) Documents cités:
- FR-A- 2 283 223
- FR-A- 2 693 654
- GB-A- 2 034 687
- STN INTERNATIONAL, KARLSRUHE, XP002024382 & AN R ACAD FARM 41 (4). 1975 541-590,
- CLIN. TER., vol. 131, no. 6, 1989, pages 413-419, XP002024383 F. GROSSI: "Riflessioni in tema di mezzi termali solfurei e dermatologia estetica"
- J. MED. BORD., vol. 144, no. 3, 1967, pages 417-426, XP002024384 R. BAUDRIMONT: "Contribution à l'étude des Diatomées des sources sulfurées chlorurées sodiques mésothermales de Saint-Saveur"

## Description

La présente invention a pour objet l'utilisation d'extraits de bactéries filamenteuses non photosynthétiques et non fructifiantes comme agents cosmétiques contre le vieillissement cutané.

On sait que le vieillissement cutané, qu'il soit dû à l'âge ou à d'autres facteurs tels que les facteurs d'environnement, se traduit notamment par une détérioration des propriétés mécaniques de la peau, et en particulier une perte d'élasticité et de tonicité, avec apparition de rides. On rattache ce phénomène notamment à une altération des tissus élastiques, et en particulier à une diminution du nombre et du diamètre des fibres élastiques.

Le vieillissement cutané s'accompagne également d'un amincissement de l'ensemble des composants de la peau, avec pour conséquence une augmentation de la fragilité cutanée. La raréfaction des fibroblastes, ainsi que l'altération de leur activité, sont considérées comme jouant un rôle très important dans le processus de vieillissement cutané.

On sait également que des échanges gazeux se produisent à la surface de la peau, avec élimination de dioxyde de carbone et absorption d'oxygène. Ce phénomène, appelé respiration cutanée, diminue avec l'âge, et cette diminution est considérée comme une résultante de la diminution de l'activité épidermique.

Le document GB-A-2 034 687 décrit la production d'une substance à activité bactériostatique dans la biomasse et/ou dans le milieu de culture d'une culture de micro-organismes du type *Beggiatoa.* Cette substance bactériostatique peut être utilisée pour les massages de la peau ou comme ingrédient pour produits cosmétiques.

Le document FR-A-2 283 223 décrit l'utilisation de la biomasse obtenue par culture de Sulfuraires de la famille des *Beggiatoacées*, ou d'extraits de cette biomasse, dans des compositions pour l'application topique telles que des pommades, des crèmes ou des laits de toilette , des laits démaquillants ou des lotions capillaires. Ces biomasses ou extraits ont notamment un effet anti-inflammatoire local et cicatrisant, ainsi qu'un effet favorable sur l'hydratation cutanée.

On a maintenant découvert que des extraits de bactéries filamenteuses non photosynthétiques et non fructifiantes sont capables de diminuer et/ou de retarder le vieillissement cutané lorsqu'ils sont appliqués sur la peau.

Ces extraits ont également un effet de stimulation sur les mélanocytes et sont susceptibles, lorsqu'ils sont appliqués sur le cuir chevelu, de diminuer et/ou de retarder le blanchiment des cheveux qui peut être considéré comme l'une des conséquences du vieillissement cutané.

Dans la présente demande, l'expression "extraits bactériens" englobe aussi bien des extraits proprement dits que les biomasses obtenues après culture des bactéries. Si désiré, ces biomasses peuvent être au moins partiellement déshydratées et/ou broyées. Bien entendu, l'invention s'étend à l'utilisation d'extraits comprenant toute fraction de la biomasse qui possède les mêmes propriétés anti-vieillissement cutané que la biomasse entière. Les extraits utilisés selon l'invention comprennent également des dérivés obtenus à partir de la biomasse, par exemple des dérivés d'acylation.

Les extraits bactériens de l'invention sont des extraits de bactéries choisies parmi les bactéries filamenteuses non photosynthétiques et non fructifiantes telles que définies selon la classification du Bergey's Manual of Systematic Bacteriology, Vol. 3 Section 23, 9e Ed., 1989.

Parmi ces bactéries, on citera plus particulièrement les bactéries appartenant à l'ordre des Beggiatoales, et notamment les bactéries appartenant au genre Beggiatoa, telles que par exemple diverses souches de Beggiatoa alba suivant la définition donnée dans Arch. Microbiol. (1984) 137, 139-144. Il convient de noter que cette définition de B.alba correspond aux anciennes appellations Beggiatoa arachnoidea, B.gigantea, B.leptomiformis, B.minima, B.mirabilis du Bergey's manual, 8e édition.

On peut citer par ailleurs les bactéries appartenant au genre Vitreoscilla (dont on sait qu'il est proche et souvent difficilement discernable du genre Beggiatoa), et aussi les bactéries appartenant aux genres Flexithrix et Leucothrix.

Les bactéries qui viennent d'être définies, et dont plusieurs ont déjà été décrites, ont généralement un habitat aquatique, et peuvent être trouvées notamment dans des eaux marines ou dans les sources d'eau thermale.

Parmi les bactéries utilisables, on peut citer par exemple :
- Vitreoscilla beggiatoïdes (ATCC 43181),
- Beggiatoa alba (ATCC 33555),
- Flexithrix dorotheae (ATCC 23163),
- Leucothrix mucor (ATCC 25107).

Pour préparer l'extrait bactérien de l'invention, il est possible de cultiver lesdites bactéries selon les méthodes connues, puis de séparer la biomasse obtenue. Un procédé de culture préféré est celui décrit dans le brevet FR-2 693 654.

Après culture des bactéries, on peut séparer et isoler la biomasse par diverses méthodes connues, par exemple par filtration, par séchage sur cylindre à précouche raclée, ou centrifugation et/ou lyophilisation. Une concentration préalable, par exemple à 80°C sous pression réduite, peut améliorer cette séparation.

On peut utiliser les extraits bactériens sous la forme de dérivés, par exemple de dérivés au moins partiellement acylés. On effectue l'acylation à l'aide d'un anhydride d'acide carboxylique, ou avec un chlorure d'acide correspondant. On peut utiliser par exemple l'anhydride acétique ou le chlorure d'acétyle. On effectue la réaction d'acylation de façon à ce qu'au moins une partie des groupements amines primaires et secondaires présents dans la biomasse bactérienne soient acylés. On détermine aisément les proportions d'agents d'acylation et les conditions de la réaction d'acylation par dosage, selon les méthodes classiques (par exemple par potentiométrie), des groupements amines, primaires et secondaires, avant et après la réaction d'acylation.

Dans les compositions utilisées selon l'invention, les extraits bactériens sont utilisés généralement dans une proportion de 0,01 à 2%, et en particulier de 0,01 à 1% en poids sec d'extrait bactérien, par rapport au poids de la composition.

Ces compositions contiennent l'extrait bactérien sous forme de dispersions dans un véhicule approprié tel que par exemple l'eau, les solvants organiques, les corps gras y compris les huiles, et leurs mélanges, notamment des émulsions.

Elles peuvent se présenter notamment sous la forme de lotions hydro-alcooliques ou oléo-alcooliques, de gels, d'émulsions de consistance liquide, de crèmes, de sticks solides ou de dispersions vésiculaires. Ces compositions peuvent être préparées selon les méthodes usuelles. Elles contiennent les ingrédients et véhicules permettant de les présenter notamment sous l'une des formes qui viennent d'être indiquées. Elles peuvent contenir en outre d'autres ingrédients actifs, tels que par exemple des substances absorbant l'ultra-violet, des agents hydratants, des agents anti-radicaux libres, des émollients, des séquestrants. Elles peuvent également contenir des ingrédients usuels tels que des agents conservateurs et des parfums.

Pour préparer des compositions sous la forme de dispersions vésiculaires à l'aide de lipides amphiphiles ioniques ou non-ioniques, on peut opérer selon les méthodes connues, par exemple en faisant gonfler les lipides dans une solutions aqueuses pour former des sphérules dispersées dans le milieu aqueux, comme décrit dans l'article de Bangham et al., J. Mol. Biol.13, 238 (1965) ou dans les brevets FR-2.315.991 et 2.416.008. D'autres modes de préparation de ces dispersions vésiculaires sont décrits dans l'ouvrage intitulé "Les liposomes en biologie cellulaire et pharmacologie" Ed.INSERM/John Libbery Eurotext, 1987, pp.6 à 18.

L'invention a en outre pour objet un procédé de traitement cosmétique pour lutter contre le vieillissement cutané, caractérisé par le fait que l'on applique sur la peau ou le cuir chevelu un extrait bactérien tel que défini ci-dessus notamment sous la forme d'une composition telle que définie précédemment.

Les compositions sont appliquées sur la peau et/ou le cuir chevelu selon les méthodes usuelles.

Les exemples suivants illustrent l'invention sans toutefois la limiter.

### EXEMPLE 1 : Sélection/adaptation et culture d'une bactérie filamenteuse non photo-synthétique et non fructifiante

On a opéré comme décrit à l'exemple 1 de FR-2.693.654.

La biomasse est récoltée par centrifugation.

On opère dans une centrifugeuse de type industriel refroidie à 20°C et capable d'imprimer une accélération supérieure à 5000 x g.

La biomasse ainsi obtenue peut être stabilisée par chauffage (par exemple à l'autoclave à 121°C pendant 20 min), par séchage, par lyophilisation ou par congélation.

### EXEMPLE 2 : Préparation d'un dérivé acétylé

A 100g de biomasse bactérienne sèche, on ajoute 155ml d'une solution d'hydroxyde de sodium à 32%.

On agite le mélange obtenu à 4°C pendant 2 heures. On ajoute alors 117 ml d'anhydride acétique.

On effectue ensuite une lyophilisation.

Par dosage des amines primaires et secondaires, avant et après acétylation, par potentiométrie avec l'acide perchlorique, on constate qu'environ 60 % des groupements amine primaires et secondaires du produit de départ sont acétylés.

De façon analogue, on a préparé des extraits bactériens acétylés au départ de biomasses bactériennes provenant de cultures de Beggiatoa alba (ATCC 33555).

### EXEMPLE 3 : Crème

Cette crème répond à la composition suivante
- Lyophilisat obtenu à l'exemple 1 0,05 %
- Carbomer 940* 0,30 %
- Triéthanolamine 0,30 %
- Acide stéarique 3,00 %
- Alcool cétylique 2,00 %
- Monostéarate de glycérol autoémulsionnable 3,00 %
- Huile de soja 10,00 %
- Alcool de lanoline 2,00 %
- Myristate d'isopropyle 4,00 %
- 2-éthylhexanoate de cétyle et de stéaryle 4,00 %
- Perhydrosqualène 3,00 %
- Paraffine 2,00 %
- Glycérine 3,00 %
- Conservateurs 0,30 %
- Eau qsp 100,00 %
*Carbomer 940 : marque de commerce désignant un acide polyacrylique réticulé.

Pour préparer cette crème, on chauffe la phase aqueuse contenant la glycérine, les conservateurs et l'eau à 80°C; on y disperse le Carbomer 940 qui est ensuite neutralisé par la triéthanolamine. La phase grasse, chauffée et homogénéisée à 80°C, est introduite sous vive agitation dans la phase aqueuse. Le lyophilisat de l'exemple 1 est dispersé dans 10 g d'eau et introduit à 40°C dans la crème, sous agitation. L'ensemble est refroidi jusqu'à température ambiante.

Cette crème est appliquée sur la peau du visage et du cou une à deux fois par jour. Elle permet notamment, après utilisation pendant un temps suffisant, d'obtenir un aspect de peau plus jeune.

### EXEMPLE 4 : Lait pour la peau

Ce lait a la composition suivante :
- Dérivé acétylé lyophilisé de l'exemple 2 0,1 %
- Monostéarate de glycérol autoémulsionnable 3,0 %
- Vaseline 1,5 %
- Huile de vaseline 2,5 %
- Huile de son de riz 1,5 %
- Huile de silicone volatile 5,0 %
- Beurre de karité 3,0 %
- Carbomer 940 0,2 %
- Triéthanolamine 0,2 %
- Gomme de xanthane 0,1 %
- Glycérine 3,0 %
- Parfum 0,1 %
- Conservateurs 0,3 %
- Eau qsp 100,0 %

Ce lait est préparé d'une façon analogue à celle décrite à l'exemple 3.

Appliqué sur la peau du visage, ce lait diminue l'effet de vieillissement cutané accéléré observé notamment chez les personnes s'exposant au soleil.

### EXEMPLE 5 : Composition à appliquer sur le cuir chevelu

Ces compositions sont destinées à retarder l'apparition des cheveux blancs.

### a) Gel

Ce gel a la composition suivante:
- Suspension congelée de l'exemple 1 5 % en matière active 1,0 %
- Carbomer 940 0,5 %
- Triéthanolamine 0,5 %
- Propylèneglycol 3,0 %
- Conservateurs 0,3 %
- Ethanol 28,0 %
- Eau qsp 100,0 %

On conseille une application par jour.

### b) Suspension hydroalcoolique

Cette suspension contient 0,5 % du dérivé acétylé lyophilisé obtenu selon l'exemple 2 dans un mélange éthanol-eau à 35 % en poids d'éthanol.

### EXEMPLE 6 : Crème

On a préparé selon le même mode opératoire qu'à l'exemple 3 une émulsion ayant la composition suivante :

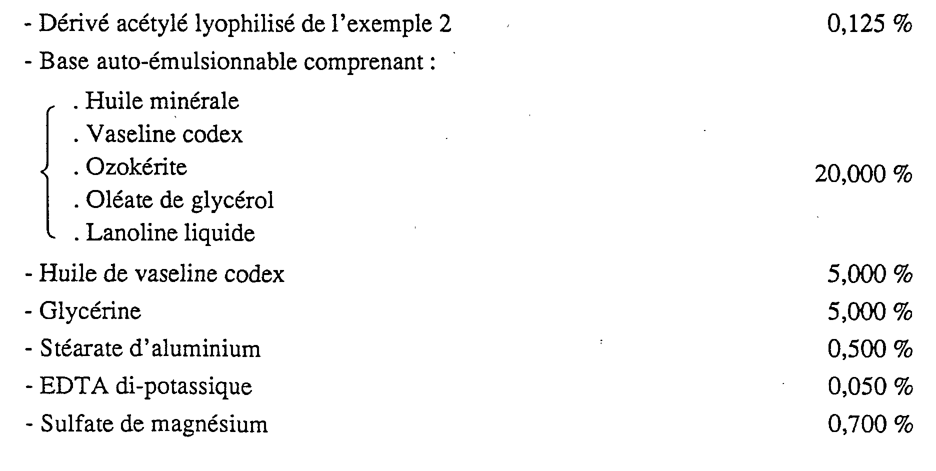

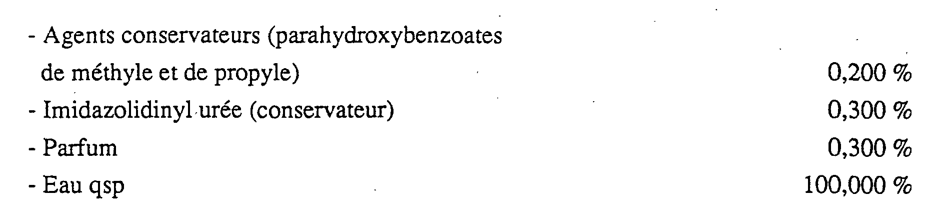

### Test de tolérance cutanée

On opère selon le test de Marzulli et Maibach: l'application répétée d'un produit ou d'une substance sensibilisante sous pansement occlusif (pour faciliter la pénétration) pendant une période 3 semaines, permet, si le produit est sensibilisant, d'induire la formation d'anticorps spécifiques.

Après une période de repos de 2 semaines, le produit est réappliqué (épreuve déclenchante).

S'il y a eu sensibilisation, une réaction allergique apparait.

### Protocole

Le test a été réalisé à l'aide de la crème préparée ci-dessus, sur 50 sujets volontaires, de la façon suivante :

### Matériel

Le produit est appliqué sous pansement occlusif de marque Leukotest dans lequel la compresse en coton recevant le produit à tester est isolée de la masse adhésive hypoallergénique par une feuille en fibre synthétique et délimitée par un anneau de cellophane permettant de limiter strictement la réaction de la peau à la surface testée.

### Application du produit

Les patches sont appliqués sur peau saine, sans préparation préalable.

Les applications sont répétées sur le même site au niveau de l'épaule gauche des volontaires, pendant toute la phase d'induction ; la même surface de peau reçoit théoriquement ainsi 3 fois par semaine, un jour sur deux, pendant trois semaines de suite, 0,1 ml du produit maintenu sous pansement occlusif pendant 48 heures ; l'application est suspendue jusqu'à l'épreuve déclenchante dès qu'il est constaté un signe d'intolérance.

Après deux semaines de repos, on procède à une dernière application du produit, d'une part sur le même site ayant servi à l'induction (épaule gauche), et d'autre part sur un nouveau site défini sur l'épaule droite de chaque volontaire : les pansements occlusifs sont maintenus en place pendant 24 heures.

### Examens

### Au cours de la phase d'induction :

Avant chaque application, on procède à une évaluation de l'état de la peau. Cet examen est renouvelé immédiatement après l'enlèvement des patches.

En cas de réaction inflammatoire éventuelle, les applications du produit doivent être suspendues, pour le sujet considéré, jusqu'à l'épreuve déclenchante.

### Lors de la phase déclenchante :

Les lectures sont effectuées immédiatement, puis 24 et 48 heures après l'enlèvement du patch.

Ces examens permettent d'observer éventuellement :
- au cours des 9 premières applications sur le même site de peau, les signes d'intolérance par accumulation du produit ;
- après l'application déclenchante, les manifestations indicatives d'un processus allergique.

### Echelle d'évaluation

Le clinicien est tenu d'apprécier l'importance des réactions de la peau selon les échelles suivantes :

Erythème :
0 - absence d'érythème
1 - érythème discret
2 - érythème franc
3 - érythème franc avec oedème

Prurit :
0 - absence de prurit
1 - prurit léger
2 - prurit important

Lésions eczématiformes :
0 - absence de vésicule
1 - présence de vésicules

Il note parallèlement les éventuelles remarques des volontaires.

### Expression des résultats

Les réactions au produit sont évaluées selon :
- un critère général qui tient compte du pourcentage de sujets présentant des réactions cutanées, quel qu'en soit le type ;
- un critère plus spécifique qui tient compte du type des réactions observées et de leur importance.

A partir de ces éléments, on définit :
- l'importance du pouvoir irritant qui se caractérise par l'apparition de réactions limitées à des érythèmes associés ou non à des oedèmes ou à un prurit ;
- l'importance du pouvoir sensibilisant qui s'exprime par des réactions érythémateuses, accompagnées généralement de prurit, d'oedème et/ou d'eczéma, observées plus particulièrement au cours de la phase déclenchante.

### Chronologie de l'essai

La chronologie des applications est la suivante :
J 0 - Première application de patch pendant 48 heures
J 2 - Lecture du résultat et application du 2^{ème} patch
J 4 - Lecture du résultat et application du 3^{ème} patch
J 7 - Lecture du résultat et application du 4^{ème} patch
J 9 - Lecture du résultat et application du 5^{ème} patch
J 11 - Lecture du résultat et application du 6^{ème} patch
J 14 - Lecture du résultat et application du 7^{ème} patch
J 16 - Lecture du résultat et application du 8^{ème} patch
J 18 - Lecture du résultat et application du 9^{ème} patch
J 21 - lecture du résultat
J 35 - dernière application du produit maintenu sous patch pendant 24 heures
J 36 - lecture du résultat 1/2 heure après l'enlèvement du patch
J 37 - lecture du résultat 24 heures après l'enlèvement du patch
J 38 - lecture du résultat 48 heures après l'enlèvement du patch.

### Résultat

### Phase d'induction

Sur 50 sujets volontaires, aucune réaction cutanée n'a été décelée.

### Epreuve déclenchante

Sur 50 sujets volontaires, aucune réaction cutanée n'a été observée.

Ces résultats sont particulièrement remarquables, compte-tenu de la présence, dans la crème testée, d'agents conservateurs et de parfum dont les risques de potentialisation des effets des substances sensibilisantes sont bien connus.

### EXEMPLE 7 : Etude de la respiration cutanée par la méthode des échanges gazeux

Le principe du test consiste à mesurer les flux d'oxygène et de dioxyde de carbone traversant la barrière épidermique, en un temps donné, sur des sujets au repos.

Pour cela on a réalisé par moulage une cellule en résine de silicone de forme cylindrique et de section circulaire. La cellule est appliquée du côté de son extrémité ouverte, sur la peau de l'avant-bras, et maintenue à l'aide d'une sangle élastique.

Le produit à tester (constitué du lyophilisat de l'exemple 1 à 0,05 %) est appliqué à raison de 2 mg/cm².

Le volume d'air emprisonné dans la cellule ainsi mise en place est d'environ 8,8 cm³ et la surface de la peau en contact avec l'air ainsi emprisonné est de 12,6 cm².

Au temps zéro, deux cellules sont fixées simultanément sur la partie à étudier, l'une recouvrant une surface de peau témoin, l'autre recouvrant une surface de peau traitée.

Un échantillon de 0,2 ml d'air est prélevé toutes les dix minutes dans la cellule à l'aide d'une seringue à gaz. La déformabilité de la cellule permet de garder une pression constante à l'intérieur de celle-ci malgré le volume d'air prélevé.

L'air prélevé est analysé par chromatographie en phase gazeuse.

Le test a été effectué sur 16 personnes d'âge compris entré 20 et 54 ans (moyenne d'âge 38 ans).

On a observé pour les zones de peau traitées, par rapport aux zones témoin non traitées, une augmentation moyenne de 27 % du flux de dioxyde de carbone et une augmentation de 21 % du flux d'oxygène à travers la peau.

### EXEMPLE 8 : Effets sur la prolifération des fibroblastes de peau humaine

Les fibroblastes sont mis en culture dans un milieu nutritif complet, à raison de 7.500 cellules par puits.

Le produit étudié est celui de l'exemple 2.

Il est ajouté dans les cultures à diverses concentrations. On procède au comptage des cellules après divers temps de culture.

On constate par exemple qu'au bout de 72 heures, le nombre de cellules traitées par 50 µg/ml du produit étudié est augmenté de 30 % par rapport au nombre de cellules des puits témoins.

Dans les surnageants de culture, on a dosé, par la technique ELISA, l'interleukine-1β, dont on sait qu'elle active le gène de l'élastine (qui est à l'origine de l'élasticité de la peau).

Dans le cas des cellules traitées à la dose de 150 µg/ml, on trouve une concentration en IL-1β de 34,5 pg/ml (témoin : 5,5 pg/ml).

Par ailleurs, la recherche d'une éventuelle activité de type élastase dans les milieux de culture (témoins et traitées) a été négative.

### EXEMPLE 9 : Essais inhibiteurs sur l'élastase

Le produit utilisé est celui de l'exemple 1 sous forme lyophilisée.

On mesure l'activité d'une élastase pancréatique (Sigma ; 83 U/mg; protéines : 15 mg/ml) en présence et en l'absence du produit étudié.

Pour cela, l'élastase (diluée à 0,15 mg protéine/ml) est ajoutée au produit additionné d'une solution de triéthanolamique (Triton X100).

On observe une diminution de 38 % de l'activité de l'élastase.

Lorsque le produit étudié est dilué de moitié, la diminution de l'activité de l'élastase est de 31 %.

### EXEMPLE 10 : Tests d'application pendant un mois sur sujets volontaires

Le produit testé est appliqué sous la forme d'une crème à 0,05 % de produit actif. Cette crème a la formulation décrite à l'exemple 3.

Le produit testé est appliqué une fois par jour à raison de 2 mg par centimètre carré de peau, sur la peau du visage et des avants-bras. La durée d'application est de quatre semaines.

Le traitement a été effectué sur dix-sept personnes volontaires.

### 1°) Mesure des propriétés mécaniques de l'épiderme

La mesure des propriétés biomécaniques de l'épiderme (extensibilité Ue, tonicité Ur, élasticité Ur/Ue) est réalisée selon la méthode décrite par C. Escoffier et al. J. Invest. Dermatol. 93 353-357 (1989), avec l'appareil décrit par de Rigal et Lévêque, Bioeng. Skin 1 : 13-23 (1985).

Les mesures sont réalisées sur la peau de l'avant-bras par apposition de la sonde de mesure sur la peau à l'aide d'un adhésif.

Les mesures ont été effectuées au temps zéro, et au bout de deux semaines et de quatre semaines.

En ce qui concerne l'extensibilité Ue, on notait une augmentation de 23 % au bout de deux semaines (30 % à quatre semaines). Pour la tonicité Ur, l'augmentation est de 40 % dans le même temps (43 % à quatre semaines).

Il en résulte que le produit appliqué améliore les propriétés biomécaniques de l'épiderme, et en particulier l'élasticité, de façon notable, déjà au bout de deux semaines de traitement.

### 2°) Effet sur les rides

L'effet sur les rides a été étudié par analyse d'images. La méthode consiste à éclairer la réplique négative (empreinte en silicone des rides de la zone voisine de la paupière appelée "patte d'oie", à l'aide d'une lumière rasante qui engendre la formation d'ombres portées derrière chaque ride. Ces ombres sont quantifiées par analyse d'image selon la méthode décrite par Corcuff et al., J. of the Soc. of Cosmet. Chem. 34, 177-190 (1983) et par Corcuff, Acta Stereologica 2(1) 85-88 (1983).

Cette méthode permet de mesurer la surface des rides étudiées. On a trouvé que la surface des rides est significativement plus faible au bout de quatre semaines. Parallèlement, on note une diminution du nombre des rides de longueur comprise entre 1 et 9 mm, ainsi qu'une diminution de la longueur moyenne des rides de longueur inférieure à 1 mm.

### 3°) Epaisseur de la peau

On a également évalué l'effet sur l'épaisseur de la peau de l'avant bras dans la méthode dite de mesure du pli cutané (référence : Dykes et al., Arch. Dermatol. Res. 256-261, 1976), à l'aide d'une pince équipée d'un cadran gradué, commercialisée par Schnelltaster. L'épaisseur de la peau saisie entre les deux mors de la pince est lue directement sur le cadran.

On observe une augmentation significative de 2 % après deux semaines, et de 3,5 % après quatre semaines de traitement.

## Revendications

1. Utilisation d'un extrait d'au moins une bactérie filamenteuse non photosynthétique et non fructifiante comme agent cosmétique contre le vieillissement cutané.

2. Utilisation selon la revendication 1, **caractérisée par le fait que** ladite bactérie appartient à l'ordre des Beggiatoales.

3. Utilisation selon la revendication 1, **caractérisée par le fait que** ladite bactérie appartient au genre Beggiatoa, Vitreoscilla, Flexithrix ou Leucothrix.

4. Utilisation selon l'une quelconque des revendications précédentes, **caractérisée par le fait que** ladite bactérie est choisie parmi des souches de Beggiatoa alba.

5. Utilisation selon l'une quelconque des revendications précédentes, **caractérisée par le fait que** ledit extrait est constitué par la biomasse bactérienne obtenue après culture de la bactérie, ladite biomasse étant éventuellement déshydratée au moins partiellement ou lyophilisée et/ou broyée.

6. Utilisation selon l'une quelconque des revendications 1 à 4, **caractérisée par le fait que** ledit extrait est sous la forme d'un dérivé d'acylation.

7. Utilisation selon l'une quelconque des revendications précédentes, **caractérisée par le fait que** ledit extrait bactérien est appliqué sous la forme d'une composition contenant une proportion de 0,01 à 2 %, et en particulier de 0,01 à 1 % en poids d'extrait sec bactérien par rapport au poids total de la composition.

8. Utilisation selon l'une quelconque des revendications précédentes, **caracterisée par le fait que** l'on applique sur la peau ou sur le cuir chevelu ledit extrait d'au moins une bacterie filamenteuse non photosynthétique et non fructifiante.

## Patentansprüche

1. Verwendung eines Extrakts mindestens eines filamenteusen, nicht photosynthetischen und nicht fruktifizierenden Bakteriums als kosmetisches Mittel gegen die Hautalterung.

2. Verwendung gemäß Anspruch 1, **dadurch gekennzeichnet, dass** das besagte Bakterium der Ordnung der Beggiatoales angehört.

3. Verwendung gemäß Anspruch 1, **dadurch gekennzeichnet, dass** das besagte Bakterium der Gattung Beggiatoa, Vitreoscilla, Flexithrix oder Leucothrix angehört.

4. Verwendung gemäß einem der vorstehenden Ansprüche, **dadurch gekennzeichnet, dass** das besagte Bakterium ausgewählt ist unter den Stämmen von Beggiatoa alba.

5. Verwendung gemäß einem der vorstehenden Ansprüche, **dadurch gekennzeichnet, dass** der Extrakt aus der bakteriellen Biomasse besteht, erhalten nach Kultur des Bakteriums, wobei die Biomasse gegebenenfalls mindestens teilweise dehydriert oder lyophilisiert und/oder zerkleinert ist.

6. Verwendung gemäß einem der Ansprüche 1-4, **dadurch gekennzeichnet, dass** der Extrakt in Form eines Derivats der Acylierung vorliegt.

7. Verwendung nach einem der vorstehenden Ansprüche, **dadurch gekennzeichnet, dass** der Bakterienextrakt in Form einer Zubereitung angewandt wird, enthaltend eine Menge von 0,01 - 2 % und insbesondere 0,01 - 1 Gew.-% des trockenen Bakterienextrakts, bezogen auf das Gesamtgewicht der Zubereitung.

8. Verwendung gemäß einem der vorstehenden Ansprüche, **dadurch gekennzeichnet, dass** man auf die Haut oder Kopfhaut den Extrakt mindestens eines filamenteusen, nicht photosynthetischen und nicht fruktifizierenden Bakteriums aufbringt.

## Claims

1. Use of an extract of at least one non-photosynthetic and non-fruiting filamentous bacterium as a cosmetic agent against skin ageing.

2. Use according to Claim 1, **characterized in that** the said bacterium belongs to the order of the Beggiatoales.

3. Use according to Claim 1, **characterized in that** the said bacterium belongs to the genus Beggiatoa, Vitreoscilla, Flexithrix or Leucothrix.

4. Use according to any one of the preceding claims, **characterized in that** the said bacterium is selected from strains of Beggiatoa alba.

5. Use according to any one of the preceding claims, **characterized in that** the said extract consists of the bacterial biomass which is obtained after culturing the bacterium, with the said biomass being, where appropriate, at least partially dehydrated, or else lyophilized and/or pulverized.

6. Use according to any one of Claims 1 to 4, **characterized in that** the said extract is in the form of an acylation derivative.

7. Use according to any one of the preceding claims, **characterized in that** the said bacterial extract is applied in the form of a composition containing a proportion of from 0.01 to 2%, in particular of from 0.01 to 1%, by weight of dry bacterial extract based on the total weight of the composition.

8. Use according to any one of the preceding claims, **characterized in that** the said extract of at least one non-photosynthetic and non-fruiting filamentous bacterium is applied to the skin or to the scalp.
